(19) Europäisches Patentamt / European Patent Office / Office européen des brevets

(11) **EP 3 009 838 B1**

(12) **EUROPEAN PATENT SPECIFICATION**

(45) Date of publication and mention
of the grant of the patent:
**21.08.2024 Bulletin 2024/34**

(21) Application number: **15190192.3**

(22) Date of filing: **16.10.2015**

(51) International Patent Classification (IPC):
**G01N 33/497** (2006.01)   **C12Q 1/04** (2006.01)
**G01N 33/62** (2006.01)   **A61B 5/08** (2006.01)
**A61B 5/00** (2006.01)

(52) Cooperative Patent Classification (CPC):
**G01N 33/497; A61B 5/082; A61B 5/412;**
**C12Q 1/04; G01N 33/62;** G01N 2333/205

(54) **A BREATH TEST FOR THE DIAGNOSIS OF HELICOBACTER PYLORI INFECTION**

ATEMTEST ZUR DIOGNOSE VON HELIOBACTER PYLORI-INFEKTION

TEST RESPIRATOIRE POUR LE DIAGNOSTIC D'UNE INFECTION À HELICOBACTER PYLORI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.10.2014 EP 14189417**

(43) Date of publication of application:
**20.04.2016 Bulletin 2016/16**

(73) Proprietor: **Infai GmbH**
**51105 Köln (DE)**

(72) Inventor: **AYGEN, Sitke**
**51105 Köln (DE)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

(56) References cited:
EP-A1- 1 685 851

• GISBERT ET AL: "C-urea breath test in the management of Helicobacterpylori infection", DIGESTIVE AND LIVER DISEASE, W.B. SAUNDERS, GB, vol. 37, no. 12, December 2005 (2005-12-01), pages 899 - 906, XP005168098, ISSN: 1590-8658, DOI: 10.1016/J.DLD.2005.09.006
• LUIGI GATTA ET AL: "Effect of Proton Pump Inhibitors and Antacid Therapy on 13C Urea Breath Tests and Stool Test for Helicobacter Pylori Infection", THE AMERICAN JOURNAL OF GASTROENTEROLOGY, vol. 99, no. 5, May 2004 (2004-05-01), pages 823 - 829, XP055159334, ISSN: 0002-9270, DOI: 10.1111/j.1572-0241.2004.30162.x
• MANA F ET AL: "The early effect of proton pump inhibitor therapy on the accuracy of the <13>C-urea breath test", DIGESTIVE AND LIVER DISEASE, W.B. SAUNDERS, GB, vol. 37, no. 1, January 2005 (2005-01-01), pages 28 - 32, XP004691289, ISSN: 1590-8658, DOI: 10.1016/J.DLD.2004.09.007
• HAIM SHIRIN ET AL: "Effect of Proton Pump Inhibitors on the Continuous Real Time C-Urea Breath Test", THE AMERICAN JOURNAL OF GASTROENTEROLOGY, vol. 98, no. 1, January 2003 (2003-01-01), pages 46 - 50, XP055159337, ISSN: 0002-9270, DOI: 10.1111/j.1572-0241.2003.07187.x

**Description**

The present invention relates to a method for the diagnosis of Helicobacter pylori infection, a diagnostic kit for performing the method as well as a diagnostic composition.

There are a number of known methods for diagnosing Helicobacter pylori infection, e.g. the upper digestive tract endoscopy, the stool antigen test (HpSA /Meridian, Milan, Italy), serological method (Pylortest EIA-G III / Orion Diagnostics, Espoo, Finland), antibody detection in urine (Otsuka Diagnostic, Frankfurt, Germany). The Helicobacter breath test INFAI (INFAI GmbH, Cologne, Germany) became the leading method. It is in commercial use with 75mg $^{13}$C-labeled urea together with up to 1g citric acid for adults and with 45mg $^{13}$C-labeled urea together with 100ml orange juice for children from the age of 3 years.

A severe disadvantage of nearly all these tests, however, is that proton pump inhibitors (PPIs) and antacid therapy disturb and give false negative results; see L. Gatta et al, in American Journal of Gastroenterology, 2004, p.823 -828.

An improvement of the Helicobacter breath test INFAI is subject matter of the EP 1 685 851 A1. There was found, that if patients taking proton pump inhibitors (PPIs) higher amounts of acids should be administered for overcompensating the activity of the PPI for a time of 10 minutes to one hour. An embodiment of the EP 1 685 851 A1 test is a breath test. In this test, the breath samples are taken prior to administration of the $^{13}$C-labeled urea and about 30 minutes after administration of the urea. The $^{13}$C content of $CO_2$ is then measured from these breath samples. A difference ($\Delta\delta$) between 00 min value and 30 min value of more than 3.2, in particular more than 4 per mille indicates a Helicobacter pylori infection.

The influence of $\Delta\delta$ on the test results has been discussed in J.P. Gisbert et al, in Digestive and Liver Disease, 37 (2005), p. 899-906.

It was now surprisingly found that the administration of a mixture of at least two acids, in particular selected from the group consisting of citric acid, malic acid and tartaric acid, and lowering the $\Delta\delta$ of 4 or more per mille - as disclosed in the prior art - to a value in the range of 2 per mille to 2,9 per mille, more particularly 2,5 per mille, the reliability of the breath test was improved.

Subject matter of the present invention is a method for diagnosing a Helicobacter pylori infection in a patient treated with proton-pump-inhibitors (PPIs) as defined in independent claim 1.

It is believed that the invention is based on the following scientific reasoning: The urease enzyme activity of helicobacter pylori in the stomach is partly depending on the local pH. Therefore, after PPI intake urease enzyme activity is reduced which is needed to metabolize $^{13}$C-urea to $^{13}CO_2$ for the breath test. Surprisingly, the application of the mixture of the acids in a rather high amount increases the urease enzyme activity for a short period of time but seems not reach the normal level.

Surprisingly, by lowering the cut-off point, it is possible to improve the method without reducing specificity.

Particularly the amount of acid is in the range of 5.5 to 6.5 g.

Typically, the acid can be administered as a solution with water, optional with compounds selected from the group of sweeteners, flavours and colorants.

The amount of $^{13}$C-labelled urea corresponds to 10 to 100 mg 99% $^{13}$C-urea. $^{13}$C-urea is commercially available in 99% purity. If this $^{13}$C-urea is used, amounts of 10 to 100 mg, preferably 75 mg are sufficient. If less pure $^{13}$C-urea is used, the amounts of urea must be correspondingly higher. If, for example the $^{13}$C content is 50%, about twice the amount is needed.

The breath test is run in the usual and well established method described in the literature and the patient instruction sheets, which are available also to the medical doctor. The samples are analyzed for example by gas isotope ratio mass spectroscopy or infrared spectrometer.

**Examples:**

Patients and Methods

53 consecutive dyspeptic patients infected with H pylori and 49 H pylori negative patients have been studied by both rapid urease test and histology.

Patients were given Nextium® (AstraZeneca, AB, Sweden), 40 mg once daily, 30 min before breakfast, every day for 30 days. They were instructed not to take antibiotics, bismuth compounds, H2 receptor antagonists or other acid-suppressive agents during the study period. Patients returned one day after the treatment of 30 days and the $^{13}$C breath test with the test meal comprising a mixture of citric, malic and tartaric acid was performed.

Analysis of $^{13}CO_2$

For understanding $\Delta\delta$, it is necessary to explain $\delta$-value and measurement unit. The analysis of $^{13}CO_2$ in breath CO2 with IRMS is always an analysis of the isotopomeric ratios of $^{13}CO_2/^{12}CO_2$ equivalent to the mass ratio of peaks m/z = 45/44

$$r_s = (I_{45}/I_{44})_{Sample} = (\text{Intensity of } ^{13}CO_2 / \text{Intensity of } ^{12}CO_2)_{Sample}$$

$$r_r = (I_{45}/I_{44})_{Reference} = (\text{Intensity of } ^{13}CO_2 / \text{Intensity of } ^{12}CO_2)_{Reference}$$

To determine the $^{13}CO_2$ amount it is necessary to compare this resulting ratio with the ratio of a reference gas with known isotopic composition (referenced to PDB - Pee Dee Belemite having a high $^{13}C$ ratio of 0.0112372). So, the initial measurement unit is defined as the relative difference of the unknown sample ratio to the reference ratio compared to the reference ratio. Due to the small values it is multiplied by 1000 and expressed as ‰ (per mille). This unit is then called delta-value:

$$\delta\ [‰] = (r_s - r_r)/r_r \times 1000$$

where $r_s$ and $r_r$ are the isotope ratios of the sample and reference, respectively.

A typical measurement for the time point 0 min is rs = 0.0109563. This provides for a delta-value of -25‰.

In case of breath test, the change of an initial delta-value over time after administration of a $^{13}C$-labelled compound (urea) is observed.

A typical measurement for a patient having an infection is rs = 0.0110125 providing a $\delta_t$ of -20‰.

Thus, calculated is the difference of post-treatment $\delta_t$ to pre-treatment $\delta_0$. It is called $\Delta\delta$.

$$\Delta\delta = \delta_t - \delta_0 \text{ , e.g.: } \Delta\delta = \delta_{(30min)} - \delta_{(0min)}$$

Cut-off point $\Delta\delta$-values are the most common values for urea breath tests (not DOB), even if some literature used DOB-values in the past. $\Delta\delta$ are calculated as following:

$$\Delta\delta = \delta_{(30min)} - \delta_{(0min)} = \delta(^{13}CO_2/^{12}CO_2)_{30min} - \delta(^{13}CO_2/^{12}CO_2)_{0min}.$$

The cutoff point can determine the breath test result (DOB):

$$\text{Cut-off point} = DOB_{30min} - DOB_{0min} = \delta_{(30min)} - \delta_{(0min)}$$

**Results**

53 positive and 49 negative patients were studied. Sensitivity, specifity and accuracy of the breath test after PPI treatment by using cut-off points of respectively 4‰ and 2.5‰ were calculated.

For the cut-off point of 4‰ the sensitivity has a value of 84,91% and with a specifity of 100% and accuracy of 91,18%.

For the cut-off point of 2.5 per mille the sensitivity has a value of 92.45% with a specifity of 97.96% and accuracy of 95.01%.

Using cut-off point of 2.5‰ and 1 day stop the medication achieved best sensitivity, specifity and accuracy for the breath test.

Table 1: 30 days PPI medication, different cut-off points 2, 2.5, 3, 4 ‰, sampling time 30 min

| Cut-Off | Sensitivity | Specifity | PPV | NPV | Accuracy |
|---------|-------------|-----------|-----|-----|----------|
| 2 ‰ | 92,45 % | 97,95 % | 98,00 % | 92,31 % | 95,10 % |
| 2.5 ‰ | 92,45 % | 97,96 % | 98,00 % | 92,31 % | 95,10 % |
| 3 ‰ | 86,79 % | 97,96 % | 97,87 % | 87,27 % | 92,16 % |
| 4 ‰ | 84,91 % | 100 % | 100 % | 85,96 % | 91,18 % |
| PPV = positive prediction value | | | | | |
| NPV = negative prediction value | | | | | |

**Conclusion**

The breath test meal (Refex® containing a mixture of organic acids) increased sensitivity and accuracy of urea breath test in patients taking PPI.

This is the first $^{13}$C urea breath test that can be used in patients regardless of PPI intake with high sensitivity, specifity and accuracy with only 1 day stop.

**References**

Gatta L, Vakil N, Ricci C, Osborn JF, Tampieri A, Perna F, Miglioli M, Vaira D. Effect of proton pump inhibitors and antacid therapy on 13C urea breath tests and stool test for Helicobacter pylori infection. Am J Gastroenterol. 2004; 99; 823-9.

European patent 1685851: A method for the diagnosis of helicobacter pylori infection and diagnostic kit for performing the method

**Claims**

1. A method for diagnosing a Helicobacter pylori infection in a patient treated with proton-pump-inhibitors (PPIs) comprising the steps of administering to the patient a mixture of citric acid, malic acid, tartaric acid in amount of 5 to 7 g, collecting a first breath sample, administering to the patient $^{13}$C-labeled urea, wherein the amount of $^{13}$C-labelled urea corresponds to 10 to 100 mg 99% $^{13}$C-urea waiting for a time of 10 to 60 minutes, thereafter collecting a second breath sample from the patient, measuring the content of $^{13}$C in the $CO_2$ of the first and second sample and determination of a $^{13}$C/$^{12}$C ratio by spectroscopy in the respective samples and

   - calculating a difference $\Delta\delta$ of the $^{13}$C/$^{12}$C ratio of the first breath sample and $^{13}$C/$^{12}$C ratio of the second breath sample, $

   wherein the method requires only a 1 day stop of PPI intake, **characterized in that** a value of the difference $\Delta\delta$ in the range of 2 per mille to 2.9 per mille is used as a cut-off to indicate the presence of a H. pylori infection in the patient.

2. The method of to claim 1 wherein the amount of acid is in the range of 5.5 to 6.5 g.

3. The method of any one of claims 1 to 2 wherein the acid is administered as a solution with water, optional with compounds selected from the group of sweeteners, flavours and colorants.

**Patentansprüche**

1. Verfahren zum Diagnostizieren einer Helicobacter-pylori-Infektion bei einem Patienten, der mit Protonenpumpenhemmern (PPIs) behandelt wird, umfassend die Schritte: Verabreichen eines Gemischs von Zitronensäure, Äpfelsäure und Weinsäure in Mengen von 5 bis 7 g an den Patienten, Sammeln einer ersten Atemprobe, Verabreichen von $^{13}$C-markiertem Harnstoff an den Patienten, wobei die Menge an $^{13}$C-markiertem Harnstoff 10 bis 100 mg 99%-igem $^{13}$C-Harnstoff entspricht, 10 bis 60 Minuten lang warten, danach Sammeln einer zweiten Atemprobe von dem Patienten, Messen des Gehalts an $^{13}$C in dem $CO_2$ der ersten und der zweiten Probe und Bestimmen eines $^{13}$C/$^{12}$C-Verhältnisses durch Spektroskopie in den jeweiligen Proben und

Berechnen einer Differenz $\Delta\delta$ zwischen dem $^{13}$C/$^{12}$C-Verhältnis der ersten Atemprobe und dem $^{13}$C/$^{12}$C-Verhältnis der zweiten Atemprobe,
wobei das Verfahren nur eine 1-tägige Unterbrechung der PPI-Einnahme erfordert,
**dadurch gekennzeichnet, dass**
ein Wert der Differenz $\Delta\delta$ im Bereich von 2 Promille bis 2,9 Promille als Grenzwert verwendet wird, der die Anwesenheit einer H.-pylori-Infektion bei dem Patienten anzeigt.

2. Verfahren gemäß Anspruch 1, wobei die Menge der Säure im Bereich von 5,5 bis 6,5 g liegt.

3. Verfahren gemäß einem der Ansprüche 1 bis 2, wobei die Säure als Lösung mit Wasser verabreicht wird, gegebenenfalls mit Verbindungen, die aus der Gruppe der Süßungsmittel, Aromen und Farbstoffe ausgewählt sind.

**Revendications**

1. Procédé de diagnostic d'une infection à Helicobacter pylori chez un patient traité par des inhibiteurs de la pompe à protons (IPP) comprenant les étapes suivantes : administration au patient d'un mélange d'acide citrique, d'acide malique et d'acide tartrique dans une quantité de 5 à 7 g, collecte d'un premier échantillon d'haleine, administration au patient d'urée marquée au $^{13}$C, dans lequel la quantité d'urée marquée au $^{13}$C correspond à 10 à 100 mg de $^{13}$C-urée à 99 %, attente pendant une durée de 10 à 60 minutes, collecte ensuite d'un deuxième échantillon d'haleine du patient, mesure de la teneur en $^{13}$C dans le $CO_2$ du premier et du deuxième échantillon et détermination d'un rapport $^{13}$C/$^{12}$C par spectroscopie dans les échantillons respectifs et

- calcul d'une différence $\Delta\delta$ entre le rapport $^{13}$C/$^{12}$C du premier échantillon d'haleine et le rapport $^{13}$C/$^{12}$C du deuxième échantillon d'haleine,
dans lequel le procédé ne nécessite qu'un arrêt de 1 jour de prise d'IPP,
**caractérisé en ce que**
une valeur de la différence $\Delta\delta$ dans la plage de 2 pour mille à 2,9 pour mille est utilisée comme seuil de coupure pour indiquer la présence d'une infection à H. pylori chez le patient.

2. Procédé selon la revendication 1 dans lequel la quantité d'acide est dans la plage de 5,5 à 6,5 g.

3. Procédé selon l'une quelconque des revendications 1 à 2, dans lequel l'acide est administré sous forme de solution avec de l'eau, facultativement avec des composés choisis dans le groupe des édulcorants, des arômes et des colorants.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1685851 A1

- EP 1685851 A

**Non-patent literature cited in the description**

- **L. GATTA et al.** *American Journal of Gastroenterology,* 2004, 823-828
- **J.P. GISBERT et al.** *Digestive and Liver Disease,* 2005, vol. 37, 899-906

- **GATTA L ; VAKIL N ; RICCI C ; OSBORN JF ; TAMPIERI A ; PERNA F ; MIGLIOLI M ; VAIRA D.** Effect of proton pump inhibitors and antacid therapy on C urea breath tests and stool test for Helicobacter pylori infection. *Am J Gastroenterol.,* 2004, vol. 99, 823-9